# EUROPEAN PATENT SPECIFICATION

(11) **EP 0 405 691 B1**
(45) Date of publication and mention of the grant of the patent: **15.02.1995**
(21) Application number: 90201705.2
(22) Date of filing: 27.06.1990
(51) Int. Cl.: C12P 41/00, C12P 17/04, C07D 317/20

(54) **A process for the preparation of a S-2,2-R1,R2-1,3-dioxolane-4-methanol**
Verfahren zur Herstellung von S-2,2-R1,R2-1,3-Dioxolan-4-methanol
Procédé de préparation de S-2,2-R1,R2-1,3-dioxolane-4-méthanol

(30) Priority: 29.06.1989 EP 89201733
(43) Date of publication of application: 02.01.1991
(73) Proprietor: SHELL INTERNATIONALE RESEARCH MAATSCHAPPIJ B.V., 2596 HR Den Haag (NL)
(72) Inventor: De Smet, Marie José, NL-3703 EK Zeist (NL); De Boer, Lex, NL-2624 KZ Delft (NL)

(56) References cited:
- EP-A- 0 244 912
- WO-A-85/03704
- CHEMISTRY LETTERS, 1984, The Chemical Society of Japan (JP); T. MUKAIYAMA et al., pp. 401-404#

## Description

The present invention relates to a process for the preparation of a 2,2-R₁,R₂-1,3-dioxolane-4-methanol consisting predominantly of or substantially completely of S-isomer.

S-2,2-R₁,R₂-1,3-dioxolane-4-methanol is an important starting material for the preparation of agricultural and pharmaceutical products, see for example J. Jurczak et al., Tetrahedron vol. 42, no. 2, pp. 447-488 (1986).

In recent years, S-2,2-R₁,R₂-1,3-dioxolane-4-methanol has become of interest as an important starting compound for the preparation of many biologically active products and especially for the preparation of chiral drugs. The use of chiral starting materials to prepare biologically active products in optically pure form is advantageous, enabling precise planning and efficient realization of synthetic pathways. S-2,2-R₁,R₂-1,3-dioxolane-4-methanol is an important example of a C₃-synthon and is used as starting compound for the preparation of many other C₃-synthons which are widely used in organic synthesis as a chiral building block. For example, S-2,2-R₁,R₂-1,3-dioxolane-4-methanol can be used in the synthesis of other chiral synthons, monosaccharides, their derivatives and other polyhydroxyl systems, and biologically active products of more complex structure.

Therefore there is still a great need for a process, usuable on an industrial scale giving economically attractive yields for the stereoselective preparation of the S-stereoisomer of 2,2-R₁,R₂-1,3-dioxolane-4-methanol. The present invention provides such a process. It has been found that the stereoselective preparation of the S-isomer can be advantageously carried out using microorganisms or substances derived therefrom. Moreover it has been found that the R-stereoisomer of 2,2-R₁,R₂-1,3-dioxolane-4-methanol may be advantageously converted into S-2,2-R₁,R₂-1,3-dioxolane-4-carboxylic acid using these microorganisms or substances derived therefrom.

European Patent Application EP-A-0244912 describes a process for the preparation of the R-isomer of 2,2-R₁,R₂-1,3-dioxolane-4-methanol starting with the racemate. According to this European patent application the S-isomer is oxidized to its corresponding acid. The enzyme system involved in the oxidation of (R) isomer is completely different from the one involved in the oxidation of (S) isomer. In general, the (R) and (S) enantiomer of a compound behave as two different substrates in enzymatic conversions.

The present invention provides a process for the preparation of 2,2-R₁,R₂-1,3-dioxolane-4-methanol enriched in S-isomer wherein R₁ and R₂ are each independently H or an alkyl group, optionally substituted or branched, or wherein R₁ and R₂ together with the carbon atom to which they are attached form a carbocyclic ring, optionally substituted, which comprises subjecting a mixture of R- and S-2,2-R₁,R₂-1,3-dioxolane-4-methanol to the action of a microorganism or substance derived therefrom which is capable of stereoselective consumption of R-2,2-R₁,R₂-1,3-dioxolane-4-methanol for a period of time such that R-2,2-R₁,R₂-1,3-dioxolane-4-methanol in the mixture is consumed to give a 2,2-R₁,R₂-1,3-dioxolane-4-methanol enriched in S-isomer. "Enriched in S-isomer" means that more than a racemic quantity of S-isomer is present. Suitably at least 50 wt% of R-2,2-R₁,R₂-1,3-dioxolane-4-methanol is consumed, to give 2,2-R₁,R₂-1,3-dioxolane-4-methanol enriched in S-isomer or consisting of substantially pure S-isomer. "Substantially pure" means that the R-isomer is consumed to such an extent that the S-isomer is 100% pure within the experimental error. The 2,2-R₁,R₂-1,3-dioxolane-4-methanol enriched in S-isomer may be at least partly isolated and/or used as starting material for the preparation of other optically active compounds. Advantageously R₁ and R₂ are each an alkyl group containing less than 6 carbon atoms or form a carbocyclic ring containing less than 8 carbon atoms. Preferably R₁ and R₂ are identical. In this way no extra asymmetry is brought in the compounds. More preferably R₁ and R₂ are each an alkyl group containing 1-3 carbon atoms or together with the carbon atom to which they are attached form a carbocyclic ring containing 5 or 6 carbon atoms.

As hereinbefore described the mixture of R and S isomers of 2,2-R₁,R₂-1,3-dioxolane-4-methanol may be converted into 2,2-R₁,R₂-1,3-dioxolane-4-methanol enriched in S-isomer. In this way only part of the mixture can be used in further reactions, for example starting with a 50 wt% R-and 50 wt% S-mixture only half of the 2,2-R₁,R₂-1,3-dioxolane-4-methanol becomes available in a useful way. According to an embodiment of the invention, R-2,2-R₁,R₂-1,3-dioxolane-4-methanol is advantageously converted into S-2,2-R₁,R₂-1,3-dioxolane-4-carboxylic acid.

In this way not only the preparation of 2,2-R₁,R₂-1,3-dioxolane-4-methanol enriched in S-isomer is possible, but at the same time 2,2-R₁,R₂-1,3-dioxolane-4-carboxylic acid enriched in S-isomer may be obtained. In this way substantially the R as well as the S-2,2-R₁,R₂-1,3-dioxolane-4-methanol may be used. The 2,2-R₁,R₂-1,3-dioxolane-4-carboxylic acid enriched in S-isomer may be used as starting compound for the preparation of many biologically active products or may be converted to the 2,2-R₁,R₂-1,3-dioxolane-4-methanol enriched in R-isomer, which is an important starting compound as well.

By the term "microorganisms capable of stereoselective consumption" is meant, for example, bacteria, yeasts, fungi. Preferably yeasts and fungi are used, more preferably microorganisms belonging to the genus Absidia, to the genus Branhamella, to the genus Graphium, the genus Trichosporon or the genus Arthrobacter can be used.

These microorganisms are selected for their ability for stereoselective consumption of R-2,2-R₁,R₂-1,3-dioxolane-4-methanol. In general microorganisms may be those which:
a. have been described in the literature as organisms capable of oxidising compounds other than the one under investigation,
b. belong to the same species as described under a., but from different strain collections and isolated from a different source,
c. belong to the same genus as those described under a., but from which it is still unknown that they can oxidize,
d. have shown to be able to grow on substrates which require a first oxidation or hydroxylation step in the degradation of these substrates.

In an embodiment of the invention the microorganisms having the ability for stereoselective consumption of R-2,2-R₁,R₂-1,3-dioxolane-4-methanol may be selected by using a R-2,2-dimethyl-1,3-dioxolane-4-methanol as the C-source in a suitable medium.

A number of microorganisms isolated from natural sources, particularly from soil samples, were selected according to the above screening procedure, and when subjected to further tests as hereinafter described, showed their ability for stereoselective oxidation of R-2,2-R₁,R₂-1,3-dioxolane-4-methanol.

The microorganisms may be isolated from different environments e.g.:
- oil spills
- areas polluted with organic chemical compounds
- waxy areas like e.g. plant surfaces
- decaying natural organic material
- natural environments in which the compound under investigation (or a related compound) may occur.

Also microorganisms, which have become capable of consumption of R-2,2-R₁,R₂-1,3-dioxolane-4-methanol through the introduction of novel genetic material are embodied by the term "microorganism, capable of stereoselective consumption".

This can be accomplished by transferring the cloned gene encoding a polypeptide responsible for the stereoselective consumption, an enzyme, from any of the screened microorganisms to another microorganism, particularly to Escherichia coli.

Other microorganisms may belong to the genus Saccharomyces, Kluyveromyces, Bacillus, Nocardia, Rhodococcus, Escherichia or Corynebacterium. Cloned genes may be selected for their ability to encode an enzyme capable of consuming R-2,2-R₁,R₂-1,3-dioxolane-4-methanol in preference to S-2,2-R₁,R₂-1,3-dioxolane-4-methanol. Alternatively they may be selected by cross-hybridization with an already selected gene encoding an enzyme for the stereoselective consumption.

The microorganisms may advantageously be immobilized, for example on a polymer gel. This can be done with growing cells, non-growing cells and/or killed cells, alternatively with suitable enzymes derived therefrom, which may be purified to a certain extent if a higher specific activity is needed.

Therefore the term "microorganisms or substances derived therefrom" means the microorganisms in a growing stage, in a non-growing stage or killed, extracts therefrom, optionally concentrated or purified. For example, enzymes optionally in combination with, for example, artifical or natural co-factors, may be used. No fermentatively active cells may be used for the consumption of the R-2,2-R₁,R₂-1,3-dioxolane-4-methanol. It is found that enzymes derived from the cells or killed cells may consume the R-isomer under suitable conditions. The microorganisms or substances derived therefrom may be used several times and are active for at least 2 weeks. Even without co-substrate (for example glucose) the microorganisms may remain active. The enrichment in S-isomer of 2,2-R₁,R₂-1,3-dioxolane-4-methanol may take place in a suitable buffer (for example 3-(N-morpholino)propane sulfonic acid, tris(hydroxymethyl)aminomethane or potassium phosphate) as well as in physiological salts. After being stored the induced cells are found to be directly capable of S-isomer enrichment.

More particularly the microorganism for the consumption of R-2,2-R₁,R₂-1,3-dioxolane-4-methanol may be a culture of Absidia glauca (a sample of this species is deposited with the CBS under the accession number of 306.89), Branhamella catarrhalis (a sample of this species is deposited with the CBS under the accession number of 307.89) or Graphium penicillioides (a sample of this species is deposited with the CBS under the accession number of 318.72), Trichosporon adeninovorans (a sample of this species is deposited with the CBS under the accession number of 8244) and Bacterium F-7 (a sample of this species is deposited with the CBS under the accession number of 238.90). Bacterium F-7 probably is a species of the genus Arthrobacter or Actinomycetes.

The S-2,2-R₁,R₂-1,3-dioxolane-4-methanol produced according to the present invention may be used as a starting material for the production of other optically active compounds. An important possibility is the production of chiral drugs.

It will be appreciated by everyone skilled in the art that any suitable process may be used to convert S and R-2,2-R₁,R₂-1,3-dioxolane-4-methanol to other optically active compounds.

Examples which illustrate the use of S and R-2,2-R₁,R₂-1,3-dioxolane-4-methanol for the preparation of optically active compounds are known from, for example, J. Jurczak et al, Tetrahedron report number 195, Tetrahedron 42 (1986), p. 447-488 and the Technical Information Bulletin 225, September 1983 of Janssen Chimica (Belgium).

The optically active compounds may be used in pharmaceutical or agricultural products.

According to a preferred embodiment of the process of the present invention a microorganism capable of stereoselective consumption of R-2,2-R₁,R₂-1,3-dioxolane-4-methanol is cultured for about 0.5 to 10 days, whereafter the cells of the microorganisms are suspended in a liquid nutrient medium and the mixture of R- and S-2,2-R₁,R₂-1,3-dioxolane-4-methanol is subjected to the action of the cells.

After the abovementioned cultivation for about 0.5 to 10 days the cells may be isolated from the culturing medium before suspending the cells in the liquid nutrient medium.

To grow the microorganism used for the selective consumption of R-2,2-R₁,R₂-1,3-dioxolane-4-methanol, ordinary culture mediums containing an assimilable carbon source (for example glucose, lactate, sucrose, etc.), an assimilable nitrogen source (for example ammonium sulphate, ammonium nitrate, ammonium chloride, etc.), with an agent for an organic nutrient source (for example yeast extract, malt extract, peptone, meat extract, etc.) and an inorganic nutrient source (for example phosphate, magnesium, potassium, zinc, iron and other metals in trace amounts) may be used.

Another preferred culture medium is a YPD-medium optionally enriched with one or more ingredients. a YPD-medium consisting of 20 g/l Bactopeptone (TM), 10 g/l yeast extract and 20 g/l glucose may be used. Before use, the medium is conveniently sterilized for 30 minutes at 110°C.

Another preferred culture medium is a skimmed milk medium optionally enriched with one or more ingredients, for example, a skimmed milk medium containing: 10% skimmed milk from skimmed milkpowder, which is conveniently sterilized for 30 minutes at 110°C before use.

Examples of enrichments to the skimmed milk medium include Maxatase (TM) (Gist-brocades).

A temperature of 0 to 45°C and a pH of 3.5 to 9 is preferably maintained during the growth of the microorganism. Preferably the microorganism is grown at a temperature of 20 to 37°C and at a pH of 5 to 9. The aerobic conditions required during the growth of the microorganisms can be provided by any of the well-established procedures, provided that the supply of oxygen is sufficient to meet the metabolic requirement of the microorganism. This is most conveniently achieved by supplying oxygen, suitably in the form of air and optionally at the same time shaking or stirring the reaction liquid. During the consumption of R-2,2-R₁,R₂-1,3-dioxolane-4-methanol by the microorganism, the microorganism might be in a growing stage using an abovementioned ordinary culture medium.

During the consumption of R-2,2-R₁,R₂-1,3-dioxolane-4-methanol by the microorganism, an ordinary culture medium may be used containing an assimilable carbon source when required (for example glucose, lactate, sucrose, etc.), an assimilable nitrogen source when required (for example ammonium sulphate, ammonium nitrate, ammonium chloride, etc.), with an organic nutrient source when required (for example yeast extract, malt extract, peptone, meat extract, etc.) and an inorganic nutrient source when required (for example phosphate, magnesium, potassium, zinc, iron and other metals in trace amounts).

Advantageously, during the enrichment of S-isomer, a BHI, a PSIII salt medium, a hydrolyzed skim milk medium, a YPD-medium (as described above) or a skimmed milk medium (as described above) optionally enriched with one or more ingredients can be used.

A BHI (brain-heart infusion) medium containing 0.037 g/l BHI (Oxoid (TM)), the pH adjusted to 7.0, can be used. Before use this medium is conveniently sterilized for 20 minutes at 120°C.

PSIII salt medium of the following composition can be used:
potassium dihydrogen phosphate (2.1 g/l),
ammonium monohydrogen phosphate (1.0 g/l),
ammonium sulphate (0.9 g/l),
potassium chloride (0.2 g/l),
sodium citrate (0.29 g/l),
calcium sulphate.2H₂O (0.005 g/l),
magnesium sulphate.7H₂O (0.2 g/l),
ammonium ferrous sulphate.6H₂O (2.5 mg/l),
zinc sulphate.7H₂O (0.5 mg/l),
manganese chloride.4H₂O (0.3 mg/l),
copper sulphate.5H₂O (0.15 mg/l),
cobalt chloride.6H₂O (0.15 mg/l),
ortho-boric acid (0.05 mg/l),
sodium molybdate.2H₂O (0.055 mg/l) and
potassium iodide (0.1 mg/l), the pH was adjusted at 6.8.
Before use the PSIII salt medium is conveniently sterilized for 30 minutes at 120°C.

Hydrolyzed skim milk medium:
Skim milk powder (Oxoid (TM)) 100 g/l, adjust to pH 7.8 and add 25 ml of a filtered solution of maxatase (40 g/l); stir the final solution at 40°C for one hour and adjust to pH 7.0. Before use the medium is conveniently sterilized for 30 minutes at 110°C.

The microorganism can be kept in the non-growing stage, for example, by exclusion of the assimilable carbon source or by exclusion of the nitrogen source. A temperature of 0 to 45°C and a pH of 3.5 to 9 can be maintained during this stage.

Preferably the microorganisms are kept at a temperature of 20 to 37°C and a pH of 5 to 9. The aerobic conditions required during this stage can be provided according to any of the well-established procedures, provided that the supply of oxygen is sufficient to meet the metabolic requirement of the microorganisms. This is most conveniently achieved by supplying oxygen, suitably in the form of air and optionally, at the same time, shaking or stirring the reaction liquid. The S- and any remaining R-2,2-R₁,R₂-1,3-dioxolane-4-methanol after transformation as mentioned above, can then be recovered and purified according to any of the procedures known per se for such products.

The microorganisms can be kept on agar slants, frozen in 50% glycerol or lyophilised. If required, pre-cultures of these microorganisms can be made according to any of the well-established procedures, for example, the microorganisms can be incubated in bouillon or in BHI (Brain Heart Infusion) for 24 hours at 30°C in a rotary shaker. A bouillon medium of the following composition can be used: Lab Lemco L 29 (meat extract, Oxoid (TM) (9 g/l), Bactopeptone (TM) (10 g/l) and sodium chloride (5 g/l), the pH adjusted to 7.6. Before use this medium is conveniently sterilized for 20 minutes at 120°C.

The present invention will be further illustrated with reference to the following Examples.

### Example 1

The microorganisms (yeasts and fungi) were grown for 24-48 hours at 26°C in 125 ml baffled flasks containing 25 ml of YPD medium. After this period the cell mass was collected by centrifugation and resuspended in 25 ml of 10% hydrolyzed skim milk medium (Hydrolyzed skim milk medium: skim milk powder (Oxoid) 100 g/l; adjust to pH 7.8 and add 25 ml of a filtered solution of Maxatase (TM) (40 g/l); stir the final solution at 40°C for one hour and adjust to pH 7.0. Sterilization: 30 minutes at 110°C). About 1.5 g/l R,S-2,2-dimethyl-1,3-dioxolane-4-methanol (racemic mixture) was added. The mixtures were shaken in 125 ml baffled flasks at 26°C for 48 hours.

The mixtures were extracted with 25 ml ethyl acetate. In this way about 40% of the 2,2-dimethyl-1,3-dioxolane-4-methanol was recovered. Hereafter, one culture of each form was taken, extracted and the remaining 2,2-R₁,R₂-1,3-dioxolane-4-methanol was analysed. The enantiomeric distribution was determined by formation of diastereoisomers with N,O-bis-(trimethylsilyl)-trifluoracetamide (BSTFA) which were separated on a chiral complexation column. The results are given in Table 1.

**Table 1**

| Microorganism | g/l* | enantiomeric ratio | |
|---|---|---|---|
| | | %R | %S |
| Absidia glauca (CBS 306.89) | 1.3 | 43 | 57 |
| Branhamella catarrhalis (CBS 307.89) | 1.9 | 33 | 67 |
| Graphium penicillioides (CBS 318.72) | 1.1 | 29 | 71 |

| | | | |
|---|---|---|---|
| * The values presented have been corrected for loss due to extraction. | | | |

### Example 2

Branhamella catarrhalis CBS 307.89 was grown for 24-48 hours at 26°C in 100 ml baffled flasks containing 25 ml of YPD medium. Hereafter the cell mass was collected by centrifugation and resuspended in 25 ml of 10% hydrolyzed skim milk medium containing 1.5 g/l R,S-2,2-dimethyl-1,3-dioxolane-4-methanol. The mixtures were shaken in 100 ml baffled flasks at 26°C for 48 hours.

The mixtures were extracted with 25 ml ethyl acetate. In this way about 40% of the 2,2-dimethyl-1,3-dioxolane-4-methanol was recovered. Hereafter, one culture of each form was taken, extracted and the remaining 2,2-dimethyl-1,3-dioxolane-4-methanol was analysed. The enantiomeric distribution was determined by formation of diastereoisomers with N,O-bis-(trimethylsilyl)-trifluoracetamide (BSTFA) which were separated on a chiral complexation column CP Cyclodex B 236 M by GC. S-2,2-dimethyl-1,3-dioxolane-4-methanol was found to cover 67% of the total 2,2-dimethyl-1,3-dioxolane-4-methanol (1.3 g/l).

### Example 3

Trichosporon adeninovorans CBS 8244 was pregrown in 100 ml baffled erlenmeyers containing 25 ml YPD medium in a shaking incubator at 30°C during 24-48 hours. 1 Ml samples of the culture were added to 100 ml erlenmeyers containing 25 ml BHI or hydrolysed skim milk medium. R,S-2,2-dimethyl-1,3-dioxolane-4-methanol was added to a final concentration of 2.0 g/l. After 48 hours of incubation the enantiomeric distribution of R,S-2,2-dimethyl-1,3-dioxolane-4-methanol was determined as described in Example 2.

| medium composition | quantity 2,2-dimethyl-1,3-dioxolane-4-methanol (g/l) | enantiomeric distribution | |
|---|---|---|---|
| | | %R | %S |
| BHI | 1.8 | 47 | 53 |
| hydrolysed skim milk | 1.8 | 45 | 55 |

### Example 4

Graphium penicillioides CBS 318.72 was pregrown on GSM medium in 100 ml baffled erlenmeyers in a shaking incubator at 30°C during 24-48 hours. 1 Ml samples of the culture were added to inoculate 25 ml BHI, PSIII and hydrolysed skim milk medium each containing 2 g/l R,S-2,2-dimethyl-1,3-dioxolane-4-methanol in 100 ml erlenmeyers. After 48 hours of incubation at 30°C the remaining R,S-2,2-dimethyl-1,3-dioxolane-4-methanol was measured as described in Example 2.

| medium composition | quantity 2,2-dimethyl-1,3-dioxolane-4-methanol (g/l) | enantiomeric distribution | |
|---|---|---|---|
| | | %R | %S |
| BHI | 1.4 | 40 | 60 |
| PSIII + 0.1% glycerol + 0.01% yeast extract | 1.6 | 48 | 52 |
| hydrolysed skim milk | 1.5 | 45 | 55 |

### Example 5

Microorganisms, capable of enantiospecific conversion of R-2,2-dimethyl-1,3-dioxolane-4-methanol into the corresponding acid from an R,S-mixture of 2,2-dimethyl-1,3-dioxolane-4-methanol, were isolated by using the following procedure. 1 Gram of a soil sample was suspended into PSIII and centrifuged. The supernatant was added to 25 ml PSIII medium containing 1 g/l R-2,2-dimethyl-1,3-dioxolane-4-methanol. The culture was incubated in a shaking incubator during 30 hours at 30°C. Dilutions were plated out on agar plates containing 1 g/l R-2,2-dimethyl-1,3-dioxolane-4-methanol and after 4 days of incubation at 30°C single cell colony isolations were carried out until monocultures were achieved. Hereafter, the isolates were precultured on BHI medium at 30°C for 24 hours and the ability for specific oxidation of the R-enantiomer in an R,S-mixture of 2,2-dimethyl-1,3-dioxolane-4-methanol was tested. 1 Ml was used to inoculate 25 ml medium in a 100 ml erlenmeyer containing PSIII, 0.1% glycerol, 0.01% yeast extract and 1.5 g/l R,S-2,2-dimethyl-1,3-dioxolane-4-methanol. After 48 hours incubation at 30°C, the remaining R,S-2,2-dimethyl-1,3-dioxolane-4-methanol was determined as described in Example 2. Using this selection procedure, microorganisms were isolated, capable of enantiospecific oxidation of the R-isomer in an R,S-mixture of 2,2-dimethyl-1,3-dioxolane-4-methanol and thus enriching the S-isomer. As example bacterium F-7 was deposited with the CBS.

### Example 6

Bacterium F-7 (CBS 238.90) was precultured in 25 ml BHI liquid medium in 100 ml shake erlenmeyers. Cells were grown during 24 hours at 30°C in a shaking incubator.

Hereafter, 1 ml of this culture was used to inoculate 25 ml medium containing PSIII, 0.1% glycerol (w/v), 0.01% yeast extract (w/v) and 1.5 g/l R,S-2,2-dimethyl-1,3-dioxolane-4-methanol. After incubation for 48 hours at 30°C, the mixtures were extracted and analysed as described in Example 2. An amount of 0.2 g/l S-enantiomer and 0.01 g/l R-enantiomer of 2,2-dimethyl-1,3-dioxolane-4-methanol was recovered, corresponding with an enantiomeric excess of 90% of the S-enantiomer.

### Example 7

Bacterium F-7 (CBS 238.90) was precultured as described before. Using the same procedure, 4 erlenmeyers (without a baffle) containing PSIII and varying R,S-2,2-dimethyl-1,3-dioxolane-4-methanol were inoculated. After 72 hours of incubation the remaining R- and S-enantiomer were determined as described before.

| initial 2,2-dimethyl 1,3-dioxolane-4-methanol (g/l) | quantity 2,2-dimethyl-1,3-dioxolane-4-methanol (g/l) | enantiomeric distribution | |
|---|---|---|---|
| | | %R | %S |
| 1.5 | 0.28 | 15 | 85 |
| 2.5 | 0.42 | 24 | 76 |
| 5.0 | 0.57 | 33 | 67 |

### Example 8

Bacterium F-7 (CBS 238.90) was pregrown on BHI medium as described in Example 5. 1 Ml of the culture was used to inoculate PSIII medium containing 2.5 g/l R,S-2,2-dimethyl-1,3-dioxolane-4-methanol and grown on a shaking incubator at 30°C during 72 hours. Hereafter, the remaining R,S-2,2-dimethyl-1,3-dioxolane-4-methanol was determined as described in Example 2 and 0.42 g/l was recovered. 76% of the R,S-2,2-dimethyl-1,3-dioxolane-4-methanol was found to be the S-isomer.

Using HPLC techniques, 2,2-dimethyl-1,3-dioxolane-4-carboxylic acid was detected. The incubation medium was treated with perchloric acid to hydrolyse the 2,2-dimethyl-1,3-dioxolane-4-carboxylic acid into aceton and glyeric acid and this was measured by HPLC using an Aminex HPX 87 H (Biorad).

Furthermore, it was investigated which enantiomer of the acid was produced. The acid was separated from the acidified incubation medium by extraction using CDCl₃. The R- and S-enantiomer were separated by the addition of naphthylethylamine and diastereoisomers were formed. Proton NMR revealed that the 2,2-dimethyl-1,3-dioxolane-4-carboxylic acid existed for 80% of the S-enantiomer.

### Example 9

Absidia glauca CBS 306.89 was pregrown in 25 ml BHI medium in 100 ml baffled flasks in a shaking incubator at 30°C during 24-48 hours. 1 ml samples of the culture were added to inoculate 25 ml BHI medium containing 1.5 g/l R,S-2,2-dimethyl-1,3-dioxolane-4-methanol. After 48 hours of incubation the enantiomeric distribution of R,S-2,2-dimethyl-1,3-dioxolane-4-methanol was determined as described in Example 2 and it was found that 55% of the R,S-2,2-dimethyl-1,3-dioxolane-4-methanol existed of the S-enantiomer.

## Claims

1. Process for the preparation of a 2,2-R₁,R₂-1,3-dioxolane-4-methanol enriched in S-isomer wherein R₁ and R₂ are each independently H or an alkyl group, optionally substituted or branched, or wherein R₁ and R₂ together with the carbon atom to which they are attached form a carbocyclic ring, optionally substituted, which comprises subjecting a mixture of R- and S-2,2-R₁,R₂-1,3-dioxolane-4-methanol to the action of a microorganism or substance derived therefrom which is capable of stereoselective consumption of R-2,2-R₁,R₂-1,3-dioxolane-4-methanol for a period of time such that R-2,2-R₁,R₂-1,3-dioxolane-4-methanol in the mixture is consumed to give a 2,2-R₁,R₂-1,3-dioxolane-4-methanol enriched in S-isomer.

2. Process according to claim 1 wherein the period of time is such that at least 50% of R-2,2-R₁,R₂-1,3-dioxolane-4-methanol is consumed.

3. Process according to claim 1 or 2 wherein the alkyl groups contain less than 6 carbon atoms or wherein the carbocyclic ring contains less than 8 carbon atoms.

4. Process according to any one of claims 1-3 wherein R₁ and R₂ are identical.

5. Process according to any of claims 1-4 wherein R₁ and R₂ are H or an alkyl group containing 1-3 carbon atoms or together with the carbon atom to which they are attached form a carbocyclic ring containing 5 or 6 carbon atoms.

6. Process according to any one of claims 1-5 wherein said microorganism is a bacterium, a yeast or a fungus belonging to the genus Absidia, to the genus Branhamella, or to the genus Graphium, to the genus Trichosporon or to the genus Arthrobacter.

7. Process according to claim 6 wherein the microorganism used is Absidia glauca, is Branhamella catarrhalis, is Graphium penicillioides, is Trichosporon adeninovorans, or is Bacterium F-7 (CBS 238.90) or mutant thereof capable of stereoselective consumption of R-2,2-R₁,R₂-1,3-dioxolane-4-methanol in which R₁ and R₂ are as defined in claim 1, in a reaction mixture containing both isomers of the 2,2-R₁,R₂-1,3-dioxolane-4-methanol derivative.

8. Process according to claim 7 wherein the microorganism used is Absidia glauca (CBS 306.89), Branhamella catarrhalis (CBS 307.89), Graphium penicillioides (CBS 318.72), Trichosporon adeninovorans (CBS 8244) or Bacterium F-7 (CBS 238.90).

9. Process according to any one of the preceding claims wherein said microorganism or substance derived therefrom is immobilized either as a growing cell, as a non-growing cell, as a killed cell, or as an enzyme.

10. Process according to any one of the preceding claims, wherein the R-2,2-R₁,R₂-1,3-dioxolane-4-methanol consumed, is substantially converted into S-2,2-R₁,R₂-1,3-dioxolane-4-carboxylic acid.

11. Process for the preparation of 2,2-R₁,R₂-1,3-dioxolane-4-methanol enriched in R-isomer comprising the conversion of S-2,2-R₁,R₂-1,3-dioxolane-4-carboxylic acid, prepared according to the process according to claim 10, into R-2,2-R₁,R₂-1,3-dioxolane-4-methanol.

12. Microorganism selected from the group consisting of
Absidia glauca (CBS 306.89),
Branhamella catarrhalis (CBS 307.89),
Bacterium F-7 (CBS 238.90)
and mutants thereof capable of stereoselective consumption of R-2,2-R₁,R₂-1,3-dioxolane-4-methanol in which R₁ and R₂ are as described in claim 1, in a reaction mixture containing both isomers of the 2,2-R₁,R₂-1,3-dioxolane-4-methanol derivative.

## Patentansprüche

1. Verfahren zur Herstellung eines an dem S-Isomer angereicherten 2,2-R₁,R₂-1,3-Dioxolan-4-methanols, worin R₁ und R₂ jeweils unabhängig für Wasserstoff oder eine gegebenenfalls substituierte oder verzweigte Alkylgruppe stehen, oder worin R₁ und R₂ zusammen mit dem Kohlenstoffatom, an das sie gebunden sind, einen gegebenenfalls substituierten carbozyklischen Ring ausbilden, welches ein Einwirkenlassen eines Mikroorganismus oder einer davon abgeleiteten Substanz, der bzw. die zum stereoselektiven Verbrauch von R-2,2-R₁,R₂-1,3-dioxolan-4-methanol befähigt ist, auf ein Gemisch von R- und S-2,2-R₁,R₂-1,3-Dioxolan-4-methanol während einer solchen Zeitdauer, daß das R-2,2-R₁,R₂-1,3-Dioxolan-4-methanol in dem Gemisch verbraucht wird, unter Bildung eines an dem S-Isomer angereicherten 2,2-R₁,R₂-1,3-Dioxolan-4-methanols umfaßt.

2. Verfahren nach Anspruch 1, worin die Zeitdauer derart ist, daß mindestens 50 % von R-2,2-R₁,R₂-1,3-Dioxolan-4-methanol verbraucht werden.

3. Verfahren nach Anspruch 1 oder 2, worin die Alkylgruppen weniger als 6 Kohlenstoffatome enthalten oder worin der carbozyklische Ring weniger als 8 Kohlenstoffatome enthält.

4. Verfahren nach einem der Ansprüche 1-3, worin R₁ und R₂ identisch sind.

5. Verfahren nach einem der Ansprüche 1-4, worin R₁ und R₂ H oder eine Alkylgruppe mit 1-3 Kohlenstoffatomen bedeuten, oder worin R₁ und R₂, zusammen mit dem Kohlenstoffatom, an das sie gebunden sind, einen 5 oder 6 Kohlenstoffatome enthaltenden carbozyklischen Ring ausbilden.

6. Verfahren nach einem der Ansprüche 1-5, worin der Mikroorganismus ein Bakterium, eine Hefe oder ein Pilz ist, die zur Gattung Absidia, Gattung Branhamella, Gattung Graphium, Gattung Trichosporon oder Gattung Arthrobacter gehören.

7. Verfahren nach Anspruch 6, worin der verwendete Mikroorganismus Absidia glauca, Branhamella catarrhalis, Graphium penicillioides, Trichosporon adeninovorans oder Bacterium F-7 (CBS 238.90) oder eine Mutante hievon ist, die zum stereoselektiven Verbrauch von R-2,2-R₁,R₂-1,3-Dioxolan-4-methanol, worin R₁ und R₂ wie in Anspruch 1 definiert sind, in einem Reaktionsgemisch befähigt sind, das beide Isomere des 2,2-R₁,R₂-1,3-Dioxolan-4-methanolderivats enthält.

8. Verfahren nach Anspruch 7, worin der verwendete Mikroorganismus Absidia glauca (CBS 306.89), Branhamella catarrhalis (CBS 307.89), Graphium penicillioides (CBS 318.72), Trichosporon adeninovorans (CBS 8244) oder Bacterium F-7 (CBS 238.90) ist.

9. Verfahren nach einem der vorstehenden Ansprüche, worin der Mikroorganismus oder die davon abgeleitete Substanz entweder als wachsende Zelle, als ruhende Zelle, als abgetötete Zelle oder als ein Enzym immobilisiert ist.

10. Verfahren nach einem der vorstehenden Ansprüche, worin das verbrauchte R-2,2-R₁,R₂-1,3-Dioxolan-4-methanol im wesentlichen in S-2,2-R₁,R₂-1,3-Dioxolan-4-carbonsäure umgewandelt wird.

11. Verfahren zur Herstellung von 2,2-R₁,R₂-1,3-Dioxolan-4-methanol, das an dem R-Isomer angereichert ist, umfassend die Umwandlung von S-2,2-R₁,R₂-1,3-Dioxolan-4-carbonsäure, die nach dem Verfahren gemäß Anspruch 10 hergestellt worden ist, in R-2,2-R₁,R₂-1,3-Dioxolan-4-methanol.

12. Mikroorganismen, ausgewählt aus der aus Absidia glauca (CBS 306.89), Branhamella catarrhalis (CBS 307.89), Bacterium F-7 (CBS 238.90) und Mutanten hievon bestehenden Gruppe, die zum stereoselektiven Verbrauch von R-2,2-R₁,R₂-1,3-Dioxolan-4-methanol, worin R₁ und R₂ wie in Anspruch 1 beschrieben sind, in einem Reaktionsgemisch befähigt sind, das beide Isomere des 2,2-R₁,R₂-1,3-Dioxolan-4-methanolderivats enthält.

## Revendications

1. Procédé pour la préparation d'un 2,2-R₁,R₂-1,3-dioxolane-4-méthanol, enrichi en isomère S, dans lequel R₁ et R₂ représentent chacun, de façon indépendante, H ou un groupe alkyle, facultativement substitué ou ramifié, ou dans lequel R₁ et R₂ conjointement avec l'atome de carbone auquel ils sont fixés forment un cycle carboxyclique, facultativement substitué, qui consiste à soumettre un mélange de R- et S-2,2-R₁,R₂-1,3-dioxolane-4-méthanol à l'action d'un microorganisme ou d'une substance qui en dérive, et qui est capable d'une consommation stéréosélective de R-2,2-R₁,R₂-1,3-dioxolane-4-méthanol pendant une période de temps telle que le R-2,2-R₁,R₂-1,3-dioxolane-4-méthanol dans le mélange est consommé pour obtenir un 2,2-R₁,R₂-1,3-dioxolane-4-méthanol enrichi en isomère S.

2. Procédé selon la revendication 1, dans lequel la période de temps est telle qu'au moins 50% de R-2,2-R₁,R₂-1,3-dioxolane-4-méthanol sont consommés.

3. Procédé selon la revendication 1 ou 2, dans lequel les groupes alkyle renferment moins de 6 atomes de carbone ou dans lequel le noyau carbocyclique renferme moins de 8 atomes de carbone.

4. Procédé selon l'une quelconque des revendications 1 à 3, dans lequel R₁ et R₂ sont identiques.

5. Procédé selon l'une quelconque des revendications 1 à 4, dans lequel R₁ et R₂ sont H ou un groupe alkyle renfermant de 1 à 3 atomes de carbone ou conjointement avec l'atome de carbone qui y sont fixés, forment un noyau carboxyclique renfermant 5 ou 6 atomes de carbone.

6. Procédé selon l'une quelconque des revendications 1 à 5, dans lequel ledit microorganisme est une bactérie, une levure ou un champignon appartenant au genre Absidia, au genre Branhamella, ou au genre Graphium, au genre Trichosporon ou au genre Arthrobacter.

7. Procédé selon la revendication 6, dans lequel le microorganisme utilisé est Absidia glauca, Branhamella catarrhalis, Graphium penicillioides, Trichosporon adeninovorans ou est un Bacterium F-7 (CBS 238.90) ou bien un mutant de ceux-ci susceptible de consommer de façon stéréosélective un R-2,2-R₁,R₂-1,3-dioxolane-4-méthanol dans lequel R₁ et R₂ sont tels que définis dans la revendication 1, dans un milieu de réaction renfermant à la fois des isomères du dérivé 2,2-R₁,R₂-1,3-dioxolane-4-méthanol.

8. Procédé selon la revendication 7, dans lequel le microorganisme utilisé est Absidia glauca (CBS 306.89), Branhamella catarrhalis (CBS 307.89), Graphium penicillioides (CBS 318.72), Trichosporon adéninovorans (CBS 8244) ou Bacterium F-7 (CBS 238.90).

9. Procédé selon l'une quelconque des revendications précédentes, dans lequel ledit microorganisme ou la substance qui en dérive est immobilisée sous la forme d'une cellule en développement, d'une cellule en non-développement, d'une cellule tuée ou d'un enzyme.

10. Procédé selon l'une quelconque des revendications précédentes, dans lequel le R-2,2-R₁,R₂-1,3-dioxolane-4-méthanol consommé, est essentiellement converti en acide S-2,2-R₁,R₂-1,3-dioxolane-4-carboxylique.

11. Procédé pour la préparation d'un 2,2-R₁,R₂-1,3-dioxolane-4-méthanol enrichi en isomère R, selon lequel on convertit l'acide S-2,2-R₁,R₂-1,3-dioxolane-4-carboxylique, préparé suivant le procédé selon la revendication 10, en R-2,2-R₁,R₂-1,3-dioxolane-4-méthanol.

12. Microorganisme choisi dans le groupe constitué par Absidia glauca (CBS 306.89), Branhamella catarrhalis (CBS 307.89), Bacterium F-7 (CBS 238.90) et les mutants de ceux-ci, susceptibles de consommer, de façon stéréosélective, le R-2,2-R₁,R₂-1,3-dioxolane-4-méthanol dans lequel R₁ et R₂ sont tels que décrits dans la revendication 1, dans un mélange de réaction qui renferme les deux isomères du dérivé 2,2-R₁,R₂-1,3-dioxolane-4-méthanol.
